(19) Europäisches Patentamt
European Patent Office
Office européen des brevets

(11) **EP 2 309 962 B1**

(12) **EUROPEAN PATENT SPECIFICATION**

(45) Date of publication and mention
of the grant of the patent:
**22.04.2020  Bulletin 2020/17**

(21) Application number: **09791125.9**

(22) Date of filing: **04.08.2009**

(51) Int Cl.:
*A61F 13/15* [(2006.01)]     *B32B 5/26* [(2006.01)]
*A61F 13/514* [(2006.01)]     *A61F 13/475* [(2006.01)]

(86) International application number:
**PCT/US2009/052639**

(87) International publication number:
**WO 2010/017158 (11.02.2010 Gazette 2010/06)**

(54) **ABSORBENT ARTICLE WITH BARRIER COMPONENT**

SAUGFÄHIGER ARTIKEL MIT BARRIERETEIL

ARTICLE ABSORBANT POURVU D'UN COMPOSANT DE PROTECTION

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **04.08.2008  US 85903 P**

(43) Date of publication of application:
**20.04.2011  Bulletin 2011/16**

(73) Proprietor: **The Procter & Gamble Company
Cincinnati, OH 45202 (US)**

(72) Inventors:
• **UDENGAARD, Brian
DK-8520 Lystrup (DK)**
• **HUMMELGAARD, Lone
DK-9000 Aalborg (DK)**

• **WISE, Brandon, Ellis
Cincinnati
OH 45208 (US)**
• **REICHARDT, Nicole, Anja
65843 Sulzbach am Taunus (DE)**
• **SPRINGOB, Christian
64653 Lorsch (DE)**

(74) Representative: **P&G Patent Germany
Procter & Gamble Service GmbH
Sulzbacher Straße 40
65824 Schwalbach am Taunus (DE)**

(56) References cited:
**WO-A1-02/12601     WO-A2-00/37723
WO-A2-02/29145     US-A1- 2006 135 923**

EP 2 309 962 B1

**Description**

FIELD OF THE INVENTION

The present disclosure is directed to an absorbent article comprising a barrier component, and may comprise a backsheet, an absorbent core and a core coversheet, said barrier component comprising a nonwoven barrier sheet, comprising at least a spunbond nonwoven web with spunbond fibers and at least a meltblown nonwoven web with meltblown fibers, said fibers being present at selected levels and said fibers having specific fiber sizes and denier, and said nonwoven barrier sheet having pores of specific maximum (or largest) pore size.

BACKGROUND OF THE INVENTION

It is well known in the art to provide absorbent articles such as sanitary napkins and diapers with a liquid barrier component, such as side flaps, leg cuffs, barrier cuffs, or for example with an anal and/ or genital cuff, also referred to as a topsheet with one or more openings. These cuffs have a high barrier function, to stop bodily exudates from wetting the skin, or from leaking out of the absorbent article.

This is for example described in co-pending application EP-A-1417945, which describes hydrophobic topsheets with one ore more openings, which have a very high alcohol repellency, in order to provide no (re)wetting of the skin by any bodily exudates stored under the topsheet.

It has been found that often materials that provide a good barrier are not very comfortable in use, because these materials may be too thick or too stiff. Examples of barrier materials comprising meltblown webs and spunbond webs are described in WO 00/37723 A2. Other meltblown webs useful in lamination are described in for example WO 02/12601 A1 and WO 02/29145 A2. The inventors found that it may be desirable that the barrier components are made of materials that not only provide an excellent barrier for urine, feces or blood, but that the barrier components are also very soft and flexible for the sensitive (baby) skin, and that they are easy to fold, easy to elasticise and wrinkle (resulting in comfortable folds or wrinkles). In some executions, it may also be desirable that a skin care composition (also referred to as lotion or lotion composition or ointment) can be applied effectively to the barrier materials, without reducing their performance. In some executions, it may also be desirable that the barrier materials can be treated (printed) with an ink composition in a durable manner.

The inventors have now found nonwoven barrier sheet and barrier components, and articles comprising it, with specific, selected meltblown and spunbond layer(s) that are such that the above performance properties are met. These nonwoven barrier sheets and barrier component comprising said sheets can be made into comfortable patterned and/ or folded barrier components, and / or comfortable elastic barrier components with comfortable wrinkles and/ or they can be effectively treated with a skin care composition and/ or an ink composition.

SUMMARY OF THE INVENTION

The present disclosure relates to an absorbent article comprising a barrier component which is part of a backsheet, or is or forms a side panel, a side flap, a waistband, a back ear or a cuff of said article and which comprises a nonwoven barrier sheet, comprising at least a spunbond nonwoven web with spunbond fibers and at least a meltblown nonwoven web with meltblown fibers, whereby said spunbond fibers have a number average fiber diameter of from 10 microns or 11 microns to 15 microns or to 14 microns, and whereby the total weight percentage (by basis weight of the nonwoven barrier sheet) of the meltblown webs (or web, if only one web is present) is from 5% to 30%, and may be from 5% to 20% or to 15%, and whereby said meltblown fibers have a number average fiber diameter from 1 micron to 5 microns or may be from 1 micron to 3 microns, said nonwoven barrier sheet has a basis weight from 10 to 45 g/ m2.

Said nonwoven barrier sheet may have pores of a maximum pore size of less than 60 microns, or may be less than 50 microns, as determined by the method set out herein. The pores may be of mean flow pore size of from 10 to 30 microns. The barrier component may have a maximum pore size within the same range as above.

It may be that said nonwoven barrier sheet comprises at least two nonwoven layers, which may be partially attached to one another. At least one of the nonwoven layers, or at least two of, or all of, the nonwoven layers may comprise said at least one meltblown nonwoven web and said at least one spunbond nonwoven web, as described herein. Hereby, or as another embodiment herein, said nonwoven barrier sheet may comprise as least 4 spunbond nonwoven webs and at least 4 meltblown nonwoven webs, and may be at least 5 meltblown nonwoven webs.

Said spunbond web may be present at least as an outer web of the barrier component and/ or nonwoven barrier sheet, i.e. at least one of the outer surfaces of the nonwoven barrier sheet and/ or barrier component is formed by a spunbond web; at least two spunbond webs may be present to form both outer webs of the nonwoven barrier sheet and/ or barrier component.

The barrier component of the absorbent article of the present disclosure provides an excellent (breathable) barrier for bodily fluids, whilst being comfortable to the skin, due to the selected fiber diameter dimensions of the spunbond fibers and meltblown fibers, and levels thereof, and the selected pore size limitations. Furthermore, the selected fibers and webs ensure that the nonwoven barrier sheet is easy to fold, ply or wrinkle, allowing easy elastication and ensuring that comfortable wrinkles or folds may be obtained.

Furthermore, the nonwoven barrier sheet and barrier component are such that allows excellent skin care composition application and transfer to the skin thereof. Even hydrophilic skin care compositions or lotions can be applied effectively, e.g. without impacting the barrier properties.

Furthermore, the barrier material is such that it allows application of ink compositions in a precise and durable manner, for example when aqueous ink compositions are applied.

The barrier component is part of a backsheet, or it is, or forms, a side panel, side flap, waistband, back ear, or cuff of said article. The barrier component may comprise a skin care composition (lotion) and/ or an ink composition. It may comprise an elastic material.

The barrier component does not require any film materials to aid the barrier properties, and thus, in one embodiment, the barrier component may be free of any non-elastic plastic film materials, or it may be free of any film materials.

## DETAILED DESCRIPTION OF THE INVENTION

As used herein, the following terms have the following meanings:
As used herein, 'absorbent article' means any article that can absorb body fluids and is suitable to be placed in close proximity to the genitals and/ or anus of the user, including in particular sanitary pads or napkins, panty-liners, or adult incontinence pads; adult incontinent briefs; adult diapers; baby, infant or toddler diaper, including so-called training pants.

As used herein 'front region' and 'back region' refer to the two regions, which are in use, respectively, closest to the front of the wearer and the back of the wearer.

When used herein, 'longitudinal' is the direction running substantially parallel to the maximum linear dimension of the component or article, and includes directions within 30° of this parallel, when applicable.

The 'lateral' or 'transverse' direction is perpendicular to said longitudinal direction and in the same plan of the majority of the article and the longitudinal axis.
As used herein, 'elastic' means typically, that the barrier component comprises elastic material, which is elastic in at least one direction, present in addition to the meltblown and spunbond layers.

As used herein, 'along' means 'at least partially substantially parallel to and adjacent to'. Adjacent includes 'in close proximity with' and 'in contact with'.

As used herein, a "nonwoven web" is a single web, whilst a "nonwoven layer" or "nonwoven laminate layer" comprises a multitude of nonwoven webs, and a "nonwoven barrier sheet" may comprise a multitude of nonwoven layers or nonwoven laminate layers (and hence a multitude of nonwoven webs).

As used herein, the term "meltblown fibers", refers to fibers formed by extruding a molten thermoplastic material through a plurality of fine, usually circular, die capillaries as molten threads or filaments into a high velocity gas (e.g., air) stream which attenuates the filaments of molten thermoplastic material to reduce their diameter to the required diameter. Thereafter, the meltblown fibers are carried by the high velocity gas stream and are deposited on a collecting surface to form a web of randomly dispersed meltblown fibers.

As used herein, the term "spunbonded fibers" refers to fibers that are formed by extruding a molten thermoplastic material as filaments from a plurality of fine, usually circular, capillaries of a spinneret with the diameter of the extruded filaments then being rapidly reduced by drawing. A spunbond nonwoven web may be produced, for example, by the conventional spunbond process wherein molten polymer is extruded into continuous filaments which are subsequently quenched, attenuated by a high velocity fluid, and collected in random arrangement on a collecting surface. After filament collection, any thermal, chemical or mechanical bonding treatment, or any combination thereof, may be used to form a bonded web such that a coherent web structure results.

The barrier component or nonwoven barrier sheet or nonwoven webs herein have the values such as number average fiber diameter, largest pore size, mean flow pore size, average fiber denier, hydro head, strike through as used herein, when said component, sheet or web has such a value on at least part thereof, as defined in the test methods set out herein. Said component, sheet or web may have said values over the whole component, sheet or web, where applicable.

Barrier component

The barrier component herein comprises a nonwoven barrier sheet. Said nonwoven barrier sheet comprises at least a spunbond nonwoven web with spunbond fibers and at least a meltblown nonwoven web with meltblown fibers. It may comprise at least 2 spunbond webs, present at, or as, the outer surfaces of said nonwoven barrier sheet. It may be that the nonwoven barrier sheet comprises at least 2, and may be at least 3 meltblown webs.

In one embodiment said nonwoven barrier sheet comprises as least 2, or at least 3 or at least 4 spunbond nonwoven webs; it may comprise at least 4 meltblown nonwoven webs, or at least 5 meltblown nonwoven webs, or at least 6 meltblown webs.

In one embodiment, the nonwoven barrier sheet is free of barrier film materials. In one embodiment it may be free of nonwoven webs other than spunbond webs and meltblown webs, e.g. free of carded webs. In one embodiment, the nonwoven barrier sheet consists of meltblown webs and spunbond webs, as described herein.

The nonwoven barrier sheet and / or barrier component may have pores of a largest pore size of less than 60 microns, or may be less than 50 microns, or may be less than 45 microns. The nonwoven barrier sheet may have pores of a narrow pore size distribution. The nonwoven barrier sheet may have pores of a mean flow pore size within the range of from 10 to 30 microns. This may be measured by the test method set out herein below.

The spunbond web has spunbond fibers with a number average fiber diameter of from 10 microns or 11 microns to 15 microns or to 14 microns. This may be measured by the test method set out herein below.

The meltblown web has meltblown fibers that have a number average fiber diameter from 1 to 5 microns, or 1 to 4 microns, or may be from 1 to 3 microns.

This is measured by the test method set out below.

The spunbond web may have spunbond fibers with an average denier of from 0.5 to 1.5 g/ 9000m, or from 0.5 to 1.2 g/ 9000m, or from 0.8 to 1.5 g/ 9000m, or from 0.8 to 1.2 g/ 9000m. As disclosed herein may be polypropylene-comprising fibers. Said meltblown fibers may have an average fiber denier of from 0.05 g/ 9000m to 0.5 g/ 9000m, may be from 0.1 g/ 9000m to 0.3 g/ 9000m. As disclosed herein may be polypropylene-comprising fibers. The denier is obtained by the test method set out below.

The total weight percentage (by weight of the nonwoven barrier sheet) of the meltblown webs (or meltblown web, if only one such web is present) is from 5% to 30%, or from 5% to 20%, or from 5% to 15%; or from 8% to 20%, or from 10% to 20%, or from 10% to 15%, by weight of the nonwoven barrier sheet.

It may be that the total weight level of meltblown webs in the nonwoven barrier sheet is 10 g/ $m^2$ or less, or 9 g/ $m^2$ or less, or 7.0 g/$m^2$ or less, or 6 g/$m^2$ or less, and/ or at least 1.5 g/$m^2$.

The nonwoven barrier sheet may have a basis weight of from 10 g/$m^2$ to 45 g/$m^2$, and may be from 15g/$m^2$ to 45g/$m^2$ or in some embodiment, to 35g/$m^2$. This can be measured by the method set out herein below.

The spunbond and meltblown fibers may be made of thermoplastic polymers, including polymer compositions, mixtures and blends. Examples of suitable thermoplastic polymers for use herein include polyolefins such as polypropylene, polyethylene, polyethylene-polypropylene copolymers, polyesters, polyamides, polyhydroxyalkanoates, aliphatic ester polycondensates, and mixtures thereof. Other suitable thermoplastic polymers include biodegradable polymers such as PHAs, PLAs, starch compositions. Polyester polymers include aliphatic containing polyesters such as poly(butylene succinate) and poly(butylene succinate adipate). Additional aliphatic containing polyesters includepoly(caprolactone), poly(ethylene succinate), poly(ethylene succinate adipate), aliphatic polyester-based polyurethanes, copolyesters of adipic acid, terephthalic acid, and 1,4-butanediol, polyester-amides, combinations and copolymers thereof, and the like.

The meltblown and/ or spunbond fibers herein may also be multicomponent fibers. A bicomponent fiber may be utilized; a bicomponent fiber may be in a side-by-side, sheath-core, segmented pie, ribbon, or islands-in-the-sea configuration. The sheath may be continuous or non-continuous around the core.

Polypropylene and polypropylene compositions may be utilized, including homopolymers of propylene, copolymers of propylene, such as, for example, block, graft, random and alternating copolymers, terpolymers, etc., and blends and modifications thereof; polypropylene homopolymers, and copolymers of propylene with ethylene and/or butane may be utilized.

In one embodiment herein, the nonwoven barrier sheet comprises at least two nonwoven layers; each comprises at least two nonwoven webs. At least one nonwoven layer, or each nonwoven layer, comprises at least a spunbond nonwoven web as described above, and at least a meltblown nonwoven web as described above. Hereby, said webs in a layer may be laminated to one another, so that the nonwoven layer may be a nonwoven laminate layer (comprising said laminated webs). Thus, in one embodiment herein, the nonwoven barrier sheet comprises at least two nonwoven laminate layers, each being laminates of nonwoven webs. At least one nonwoven laminate layer, or each nonwoven laminate layer, comprises at least a spunbond nonwoven web as described above, and at least a meltblown nonwoven web as described above (which are thus laminated).

The at least one meltblown nonwoven web and the at least one spunbond nonwoven web of the nonwoven barrier sheet, or of one layer thereof, as described herein, may be laminated to one another.

Such laminates may be formed by any number of bonding methods known to those skilled in the art including thermal bonding and adhesive bonding, sonic and ultrasonic bonding, and extrusion laminating whereby a first nonwoven web is cast directly onto a second nonwoven web or laminate, and (while still in a partially molten state) bonds to one side of said second nonwoven web; or for example by depositing melt blown fibers directly onto a second nonwoven web or laminate, to form a first nonwoven web onto said second nonwoven web.

The weight percentage of meltblown web(s) per nonwoven layer of the barrier sheet may be also as above, e.g. from 5% to 30%, or from 5% to 20%, or from 5% to 15%; or from 8% to 20%, or from 10% to 20%, or from 10% to 15%, by weight of the nonwoven layer.

In one embodiment, said two (or more) nonwoven layers or nonwoven laminate layers are not fully attached to one another, and thus not laminated to one another, may have an attachment area of 60% or less, or of 40% or less, or of 25 % or less, or of 20% or less (of the total area of overlap area between two neighbouring nonwoven layers). In one embodiment, the nonwoven layers are attached to one another along the side edges of the overlap area, e.g. along the edges of each or one of the nonwoven webs (e.g. along the periphery); the nonwoven webs may also optionally be attached to one another in the area where elastic material may be present, as described below. It may be that the nonwoven layer comprises areas, e.g. of at least 0.5 cm$^2$, where both webs are present but not attached to one another.

Herein are, for example, a nonwoven barrier sheet comprising a 22 g/m$^2$ SMMMS nonwoven laminate layer attached to another 22 g/m$^2$ SMMMS nonwoven laminate layer (whereof for example the meltblown level of each layer is 5%, as described above); or a nonwoven barrier sheet comprising a 17 g/m$^2$ SMMMS nonwoven laminate layer attached to another 17 g/m$^2$ SMMS or SMMMS nonwoven laminate layer (comprising for example 5% meltblown webs per layer, as described above); or mixtures of such nonwoven laminate layers. Hereby, all meltblown and spunbond webs of the nonwoven laminate layers (and of the barrier nonwoven sheet) may be as described herein above.

In one embodiment, the barrier component and/ or nonwoven barrier sheet may have a hydrostatic head value (measured with a 52 mN/m liquid with the hydrostatic head test set out herein) of at least 20 mbar, or at least 25 mbar, or at least 28 mbar, or at least 30 mbar, or optionally at least 35 mbar, and optionally up to 50 mbar or up to 45 mbar. A nonwoven barrier sheet or barrier component is considered to have the above hydrostatic head values if it has this value at any part of the material, excluding areas comprising elastic material or edges attached to another material: i.e. the measurement is done on a sample that does not comprise elastic material or edges attached to another material. In one embodiment, the nonwoven barrier sheet and/ or barrier component has a surface area that is free of elastics or edges of at least 2.5 cm x 2.5 cm.

The nonwoven barrier sheet and/ or barrier component has in one embodiment a surface tension strike through value, as determined by the method described herein, of at least 20 seconds, at least 30 seconds, and optionally less than 200 seconds, or less than 150 seconds or less than 100 seconds. A nonwoven barrier sheet or barrier component is considered to have the above low surface tension strike through values if it has this value at any part of the material, excluding areas comprising elastic material or edges being attached to other materials.

The barrier component may be any component of an absorbent article that needs to fulfil barrier functions, including: a backsheet component, side flap, or cuff; or for diapers, also including: a side panel, a waistband, a back ear. A barrier component herein may be part of or form a cuff. Said cuff may be a barrier cuff, or a leg cuff, or a genital cuff or an anal cuff or a genital and anal cuff, such as described herein below in more detail.

It may be that the absorbent article herein has a pair of such barrier components, e.g. a pair of back ears, a pair of side flaps or a pair of cuffs.

The barrier component may comprise, in addition to the barrier nonwoven sheet, an elastic material. The portion of the barrier component where said elastic material is present is herein referred to as elastic laminate portion.

The elastic material may be attached to a surface of the nonwoven barrier sheet that in use is not facing the skin, or that is not contacting the skin; and/ or it is attached between two of the nonwoven layers that may be present in the nonwoven barrier sheet, as described above; and/ or it is attached to the nonwoven barrier sheet and covered with an additional covering strip material, which may also be a nonwoven, so that the elastic component is not in direct contact with the skin of the user in use.

The elastic laminate portion of the barrier component herein (e.g. comprising the nonwoven barrier sheet and elastic material at least) may comprise wrinkles of an average wrinkle height of less than 800 microns, or less than 600 microns. To measure this, the elastic laminate portion is elongated (stretched) to the length that it has an elongation $\varepsilon$= 0.5, which is: 1- (contracted length/ stretched length); for example, if the contracted length is 10 cm, the laminate portion is stretched to the (partially) stretched length of 20 cm, to be elongated to $\varepsilon$= 0.5). This elastic laminate portion is examined by use of PRIMOS and its data acquisition software, following the manufacture's instructions manual, using a 13x18mm lens. This will calculate the average wrinkle height of the elastic laminate portion. (If the elastic laminate portion has an average width of more than 3 mm, then the measurement above is only done on the inner 70% of the width of the laminate portion, along its full length.)

An elastic laminate portion of the barrier component herein may be formed from a multitude of thin strands of elastic material or for example from a single band of elastic material, attached to said nonwoven barrier sheet. A barrier component that is a cuff may comprise an elastic laminate portion along part or all of the longitudinal edge, e.g. the free edge of the cuff. The width of the elastic laminate portion may vary, and may vary depending on the exact dimensions of barrier component, for example the elastic laminate portion may have an average width of about 1 mm to 40 mm, or 2 mm to 30 mm, or 2 mm, or even 3 mm to 20 mm. The elastic materials may have an average thickness (e.g. gauge) of at least 20 microns, at least 40 microns, or even at least 60 microns, up to about 300 microns, or even up to 200 microns or even up to 150 microns.

In one embodiment, the absorbent article has as a pair of barrier components herein, for example a pair of cuffs, a pair of back ears, a pair of side panels, a pair of side flaps, each comprising or being formed by said barrier component.

Each cuff may extend longitudinally along a longitudinal side edge of the backsheet and/ or the absorbent core of the article. The cuffs of a pair of cuffs may be mirror images of one another in the longitudinal axis of the absorbent article.

It may be that the cuff has a width of at least 1 cm, or at least 2 cm or at least 2.5 cm. It may be that the cuff extends along at least 50% of the total length of the article, or at least 60% or at least 70%.

The cuff may be also be a generally H-shaped cuff, having a unitary shape and having two side cuff portions joined to one another in for example the crotch region; or pair of cuffs may be attached to one another with an additional transversely extending material, such as a transverse strip, in for example the crotch region (e.g. the crotch region being the region having the centre 1/3 of the length of the article, between the back and front region if the article, each being also 1/3 of the length of the article).

In another embodiment herein, the absorbent article comprises an anal and/ or genital cuff, comprising or being formed by said barrier component; such a cuff is also referred to as topsheet with one or more openings, for the reception of bodily exudates, such as blood, urine or in particular fecal material, herein after referred to as topsheet, whereby said bodily exudates can pass through said opening or openings to a void space under the topsheet. As used herein, 'opening' (as present in the topsheet or anal and/ or genital cuff) means an area circumscribed by the topsheet, e.g. by the barrier component, but where the topsheet, e.g. barrier component, is not present, and which is large enough to receive bodily exudates, e.g. fecal material, for example being at least 2 cm long or wide, or having a surface area of at least 2 cm$^2$. As used herein, the term 'void space' is a cavity in the article present in at least the relaxed state, which serves to accept and contain bodily exudates such as fecal material, for example having a volume of at least 3 or even 5 cm$^3$ in relaxed state.

The opening may be in the form of a slit opening. The opening may be present in (part of) the front region of the topsheet (in use towards the front of the user) and in (part of) the back region. The topsheet may have a slit opening, which has a longitudinal dimension (length) substantially parallel to the longitudinal axis of the topsheet and of the diaper. It may be that (in stretched state) the opening (or openings) of the topsheet is (are) configured such that from 20% to 40%, or may be from 20% to 30% of the length of the opening (or total length of the openings) extends from the transverse axis of the topsheet towards the front edge of the topsheet, and the remaining percentage extends towards the back edge of the topsheet.

The topsheet may be formed of, or comprises, a barrier component with at least two elastic laminate portions, each along part or all of a longitudinally extending side edge of the opening or openings, said elastic laminate portions, and may be that said side edges, being mirror images of one another in the y-axis of the topsheet or article.

The topsheet may comprise a secondary elastic region in said crotch area, for example a secondary elastic region extending in longitudinal direction between a longitudinal side edge of the topsheet and the elastic laminate portion, described above, closest to said edge of the opening.

The elastic laminate portion(s) herein may be formed by attaching an elastic material in stretched state or partially stretched state to part of the barrier component. It may be attached to the outer surface of the barrier nonwoven sheet described herein, or between two nonwoven layers of said barrier nonwoven sheet; or it may be attached to one or more carrier material(s), which is (are) then subsequently attached to the barrier nonwoven sheet. In one embodiment, the elastic material is attached to the nonwoven barrier sheet described herein on the surface area of the nonwoven barrier sheet that is not in contact with the skin of the user; and/ or it is attached to the nonwoven barrier sheet with a first surface area of the elastic material and a protective sheet material is attached to the opposite surface area of the elastic material; and/ or it is attached to said nonwoven barrier sheet with a first surface area of the elastic material and then said nonwoven barrier sheet is subsequently folded, e.g. in a C-fold, over the opposite side of the elastic material, to form the elastic area.

In one embodiment, the barrier component or part thereof comprises a 3-dimensional pattern, i.e. the barrier component has a patterned area; this may be an embossed area, ringrolled area or crimped area. Due to the flexibility of the barrier component, such a pattern can be easily and precisely applied and such a patterned are can easily be obtained. It may be that only part of the barrier component comprises said pattern. It may be that the elastic laminate portion comprises such a 3-dimensional pattern, in addition to a wrinkle pattern, formed by contraction of elastics. Thus, in one embodiment the barrier component comprises a patterned elastic laminate portion.

A wrinkled, patterned elastic laminate portion of the barrier component herein, which comprises elastic material, may be obtainable by:

a) obtaining a nonwoven barrier sheet or a first barrier nonwoven layer thereof, as described herein;
b) obtaining an elastic material that is at least partially stretched, having at least an average longitudinal direction of stretch Y;
c)

i) submitting said sheet or said layer, or part thereof, to a patterning, pressure-applying step to obtain a patterned nonwoven barrier sheet or layer thereof, comprising troughs, and then positioning said at least partially stretched elastic material adjacent said patterned sheet or layer, to obtain a combined material; or

ii) positioning said at least partially stretched elastic material (15) adjacent said nonwoven barrier sheet or layer thereof, to obtain a combined material and simultaneously or subsequently submitting the combined material, or part thereof, to a patterning, pressure-applying step to obtain a patterned combined material, comprising a patterned barrier nonwoven barrier sheet or layer thereof comprising troughs (16);

d) simultaneously or subsequent to step c) attach the thus formed troughs or part thereof, of the patterned sheet or layer thereof to said elastic material;

e) optionally, if in the previous steps a nonwoven layer of the barrier nonwoven barrier sheet was attached to the elastic material, then attaching another nonwoven layer(s) to said patterned layer of the previous steps;

f) to thus obtain in step d) or e) a barrier component, comprising a patterned elastic laminate portion;

g) and then relaxing this barrier component of step f) to obtain a barrier component comprising a wrinkled, patterned elastic laminate portion, comprising wrinkles with peaks and valleys, said valleys being formed by both said elastic material and the troughs of said barrier nonwoven barrier sheet or layer thereof.

At least 10% of the elastic laminate portion may comprise said pattern of troughs, may be at least 30% or even at least 40% or even at least 60% or at least 75% or at least 90% or even about100% of its length.

At least 30% of the width of the elastic laminate portion may comprise said pattern of troughs or for example at least 50% or even at least 70% or at least 80% or at least 90% or even about 100%. Furthermore, the width of the part patterned area may be more than the width of the elastic material for example be from 100% to 500% of the average width of the elastic material, or from 105% to 250% or from 110% to 150%.

The number of troughs (along the elastic laminate portion) of the barrier component may vary; for example, it may have in contracted state an average of from 5 to 25 troughs per cm, or from 5 to 20 troughs per cm, or from 7 to 15 troughs per cm, and may be in the machine direction and direction of stretch Y.

The patterning step may be done by applying (indirectly or directly) a patterning surface of a first tool to the surface of the barrier nonwoven barrier sheet or layer thereof, and may be the surface that does not face the elastic material. This tool surface may be a continuous surface, and the tool is for example a patterned roll, with raised portions (with a first dimension x, parallel to the axis of the tool). The raised portions may have any shape, for example studs, or teeth.

The opposite surface of the nonwoven barrier sheet or layer thereof, which may face the elastic material, is pressurized and may be indirectly contacted by a surface of a second tool to apply a counter pressure to the first tool's surface; this second tool's surface may be non-mating with the first tool's surface; it may be a flat surface. The second surface may also a continuous second surface such as a surface of a second roll.

The patterning step may apply a pressure that is large enough to ensure patterning of the nonwoven barrier sheet or layer thereof and contacting of the thus formed troughs with the elastic material and possibly aiding attachment thereof to the elastic material. The applied pressure may be limited, to avoid attachment of the crests to the elastic material. Suitable pressures may depend on the nonwoven barrier sheet's properties such bending rigidity, thickness, and on the type of elastic material chemistry, thickness, and on whether for example adhesives are used.

This may for example be from 10,000 to 100,000 psi, or from 20,000 to 80.000 psi, or for example from 30,000 to 60,000 psi (obtainable by calculation). 10,000 psi correspond to 68.95 MPa.

It may be that the average distance between the highest point of the raised portions (in x-y plane) of the first tool and highest point of the surface of the second tool is from 0.01 and 1.0 mm, or from 0.025 mm to 0.6 mm or to 0.5mm or to 0.3mm or to 0.25 mm.

An example average first, length dimension (Y-direction) of the raised portions may for example be from 0.01 mm to 3 mm, or from 0.05 to 2.5 mm or to 2.0 mm, or from 0.1 to 2 mm or to 1.5 mm, or to 1 mm or to 0.5 mm; an average width dimensions (X-direction) of the teeth or studs may for example be from 0.01 mm to 5 mm, or from 0.05 to 3 mm or to 2.5 mm, or from 0.1 to 2 mm or to 1.5 mm, or to 1 mm or to 0.5 mm. The width of the ridges herein may be equal to the width of a trough of the patterned elasticized portion.

Ink composition

In one embodiment, the barrier component comprises an ink composition, may be applied in a pattern, for example by printing. The ink composition may be an aqueous composition. This can be applied to barrier nonwovens sheet or barrier component despite the presence of fibers on the surface and despite the barrier nature and may be hydrophobic nature of these barrier components and nonwoven barrier sheets herein.

The ink composition may comprise an aqueous latex component. This may for example comprise an elastomeric polymer, for example a vinyl and/ or styrene polymer, including styrene acrylic polymers and derivatives thereof and/ or styrene butadiene polymers (rubbers) and derivatives thereof. These polymers include also block polymers and copolymers.

It may comprise a surface tension reducing agent, and may be a diol surfactant. Example surface tension reducing agents may include dioctyl sodium sulfosuccinate or derivatives thereof, ethoxylated glycols, sorbitan esters and/ or acetylenic diol-based surfactants and mixtures thereof. The acetylenic diol-based surfactants may include alkoxylated (e.g..ethoxylated) acetylenic diols, including acetylenic glycols, and derivatives thereof, including alkyl branched derivatives thereof, e.g. with one or more methyl side-groups. The may for example have an alkoxylation (e.g. ethoxyaltion) degree of 2-50, and may be 4-10, or 4-8. The ink compositions may for example comprise ethoxylated 2,5,8,11 tetramethyl-6 dodecyne 5,8 diols and derivatives thereof.

The ink composition may suitably comprise at least a pigment. It may be that the pigment is present at a levels of from 0.5% to 40% by weight of the aqueous composition (prior to application), or for example from 1% to 30% by weight. It may be that the pigment is in the form of small particles, having an average particle size of less than 1 micron, or for example from 0.01 - 0.20 micron.

Skin care composition

In one embodiment herein the barrier component may comprise a skin care composition, also referred to in the art as lotion or lotion composition.

A portion of, or an entire surface of the barrier component may be coated with a skin care composition. It may be that said barrier component comprises said skin care composition (or lotion) at least on the elastic laminate portions, described above, if present. Examples of lotions include those described in U.S. 5,607,760; U.S. 5,609,587; U.S. 5,635,191; U.S. 5,643,588; WO 95/24173.

It may be a hydrophilic skin care composition, which reduces adherence of bodily exudates, such as faeces and blood.

A skin care composition may for example comprise:

> 1) first component comprising one or more liquid (at 20°C) compounds selected from the group consisting of liquid polyhydric alcoholic solvents, liquid polyethylene glycol, liquid polypropylene glycol, liquid polyethylene glycol derivatives, liquid polypropylene glycol derivatives; and liquid nonionic surfactants with HLB value of at least 10; and liquid fatty acid esters comprising at least one fatty acid unit and at least one (poly) ethylene glycol unit and/ or (poly) propylene glycol unit;
> and;
> 2) a second solid component (at 20°C) comprising one or more compounds selected from the group consisting of

>> (c) solid polyethylene glycols, solid polypropylene glycol, solid polyethylene glycol derivatives, solid liquid polypropylene glycol derivatives;
>> (e) solid nonionic surfactants with HLB value of at least 10;
>> (f) solid fatty compounds selected from the group consisting of solid fatty acids, solid fatty soaps and solid fatty alcohols.

The skin care composition may be essentially non-aqueous. Non aqueous means, that the lotion composition comprises water only in minor amounts such as less than 5 wt.% or even less than 1 wt.%, or may have no water.

Skin care compositions may include polyethylene glycols, polypropylene glycols mono- or di- end capped polyethylene glycols and mono- or di- end capped polypropylene glycols, or other polyethylene glycol derivatives, or polypropylene glycol derivatives, such as esters and ethers.

Suitable liquid alkylene or ethylene glycol fatty acid esters for skin care compositions herein are for example the esters of one or more alkylene glycol units, may be ethylene glycol units, and one or two fatty acids. Example compounds have the general formula $R^1$-$(OCH2CH2)_m$O-$R^2$ where $R^1$ and $R^2$ are hydrogen or fatty acid residues with e.g. from 6 to 30 or from 8 to 22 carbon atoms and can be the same or different with the proviso that not both are hydrogen; and m is a number of at least 1. R1 and R2 may be different and m is 1, 2, or 3. Some ethylene glycol esters are known for example as diethylene glycol diethylhexanoate/ diisononanoate, diethylene glycol diisononanoate, diethylene glycol dilaurate, diethylene glycol dioctanoate/diisononanoate and diethylene glycol distearate. Suitable trade product mixtures containing ethylene glycol esters are for example DERMOL MO or DERMOL 489. Wax esters which may be liquid at room temperature (25°C). They may be derived from natural sources such as jojoba oil, comprising docosenyl eicosenoate, eicosenyl eicosenoate and eicosenyl docosenoate.

Also useful may be EO-PO copolymers and EO-PO block copolymers, such as for example Genapol PF80, an EO-PO block copolymer from Clariant Corp.

Suitable solid nonionic surfactants with an HLB value of at least 10 include solid PEG derived nonionic surfactants, solid polyalkylene glycol fatty alcohol ethers, such as solid polyethylene glycol fatty alcohol ethers or for example solid polyethoxylated fatty alcohols. The fatty alcohols unit may have from 8 to 30 carbon atoms, may be from 12 to 22 carbon atoms. The average degree of alkoxylation, e.g. ethoxylation, may be from 2 to 200, may be at least 10, at least 20 or at least 30. These surfactants may be nonionic surfactants with HLB values of at least 10, or at least 12 or at least 13, up to for example 17. Polyethylene glycol fatty alcohol ethers have the general formula $R(OCH2CH2)_nOH$, where R represents an alkyl group or a blend of alkyl groups with for example 8 to 30 or 12 to 22 carbon atoms and n is the degree of ethoxylation, e.g. 2 to 200. Suitable PEG derived surfactants include PEG-12 stearate, PEG-100 stearate, for example available as Tego Acid S 100 P from Evonik/ Degussa.

Suitable trade products include also for example BRIJ 76, BRIJ 78 and BRIJ 700 (Steareth 100, available from Croda Inc.).

Other surfactants include Ceteraeth-10, Ceteareth-20, Polysorbate- 65. Also used may be Laureth 23.

Other compounds include PEG oils, like PEG40 hydrogenated caster oil, PEG-20 sorbitan monooleate, PEG-200 castor oil, available for example as Hetoxide C-200 from Global-Seven Inc.; glycerol esters such as a decaglycerol mono/dioleate, available for example as Caprol PGE860 from Abitec Corp.; lecithin derivatives, such as soy phosphatides, such as available as Alcolec Powder from American Lecithin Co.; sorbitan derivatives, such as Polysorbate 65, such as available as Liposorb TS-20 from Lipo Chemicals; sucrose and glucose esters and derivatives such as succinoglycan, available for example as Rheozan from Rhodia, Inc.

Exemplary skin care compositions may be such that:
said first liquid component may comprise a liquid polyethylene glycol and said second component may comprise a solid nonionic surfactant with an HLB value of at least 10, provided that when said solid nonionic surfactant is an alkoxylated (e.g. ethoxylated) fatty alcohol, then the HLB value is at least 13; or said first component may comprise a liquid fatty acid ester comprising at least one fatty acid unit and at least one ethylene glycol unit and said second component may comprise a solid polyethylene glycol; or said first component may comprise a liquid polyethylene glycol and said second compound is a solid fatty compounds selected from the group consisting of solid fatty acids and solid fatty soaps and solid fatty alcohols.

When said solid fatty compound comprises a solid fatty acid, then the total amount of liquids may be higher than the total amount of solids.

It may be that the skin care composition comprises from 20% to 80% by weight, or 30% to 70% by weight, or 40% to 60% by weight of a PEG or PPG surfactant, such as Steareth-100.

Absorbent articles

The absorbent article herein may be a sanitary napkin or pad; a panty-liner; an adult incontinent pad, brief or diaper; or an infant (baby, toddler) diaper, including training pant.

The absorbent article may comprise a liquid impervious backsheet, which may comprise the barrier component herein, or other nonwoven materials and/ or film materials as known in the art. Suitable backsheet materials may comprise breathable material, which permit vapors to escape from the diaper while still preventing exudates from passing through the backsheet. In one embodiment herein, the liquid impervious backsheet comprises a thin plastic film such as a thermoplastic film having a thickness of about 0.01 mm to about 0.05 mm. Suitable backsheet films include those manufactured by Tredegar Industries Inc. of Terre Haute, IN and sold under the trade names X15306, X10962 and X10964. It may also comprise a nonwoven sheet, attached to said film. The backsheet, or any portion thereof, may be elastically extendable in one or more directions.

The absorbent article also may comprise an absorbent core. The absorbent core may comprise any absorbent material which is generally compressible, conformable, non-irritating to the wearer's skin, and capable of absorbing and retaining urine, such as comminuted wood pulp, creped cellulose wadding; melt blown polymers, including coform; chemically stiffened, modified or cross-linked cellulosic fibers; absorbent foams; absorbent sponges; super absorbent polymers; absorbent gelling materials; or any other known absorbent material or combinations of materials; may be absorbent cores which have an absorbent storage layer which comprises more than 80% by weight of the absorbent core content (e.g. excluding core cover) of water-absorbent polymer particles or water-absorbent gelling polymers. The absorbent core or storage layer thereof may be free of absorbent cellulose fibers.

The absorbent core is may be covered by a core cover that covers at least the body-facing surface of the core, which may be in close proximity or in contact with the skin of the user. Suitable core coversheets are liquid permeable nonwovens. Also apertured films or apertured formed films may be present as core cover, may be in addition to a nonwoven core cover underlying said film.

The nonwoven webs or layers used as part of the backsheet material or core cover sheet may be webs or layers of natural fibers (e.g., wood or cotton fibers), or synthetic fibers (e.g., polyester or polypropylene or polyethylene or bicomponent fibers), or a combination of natural and synthetic fibers. The fibers may be spun bond, carded, wet-laid, melt blown, hydro entangled, or otherwise processed as is known in the art.

The absorbent article herein may be a disposable adult or infant diaper or infant training pants. The diapers herein may have a fastening system, which may be joined to the waistband, as known in the art. Example fastening systems comprise fastening tabs and landing zones, wherein the fastening tabs are attached or may be joined to the back region of the diaper and the landing zones are part of the front region of the diaper.

Test methods

Basis Weight:

The basis weight herein can be measured consistent with ASTM D 756, ISO 536 and EDANA ERT-40.3-90. It is defined as mass per unit area, in g/ $m^2$ (also referred to gsm), and measured on the component or sheet as a whole, if possible with this method, or a sample thereof; the total sample surface area may be any size suitable for the test method, but preferably a sample of 100 cm² ($\pm$ 0.5%) is used. The sample is conditioned at 23° Celsius ($\pm$ 2°C) and at a relative humidity of 50% for 2 hours to reach equilibrium, prior to weight determination.

Fiber Diameter:

The number average fiber diameters herein are determined by using a Scanning Electron Microscope (SEM) and its image analysis software. A magnification is chosen such that the fibers are suitably enlarged for measurements, e.g. between 1000 and 10,000. At least100 fibers are measured, and the number average fiber diameter is calculated with the software and used herein.

Fiber Denier:

In order to determine the average fiber denier, the number average fiber diameter is first obtained by the method above.

$$\text{Fiber denier} = \text{Cross-sectional area} * \text{density} * 9000\text{m} * 1000 \text{ g/kg.}$$

The cross-sectional area is $\pi$*diameter²/4. The density of the fibers used is known from suppliers, text books etc; for example for polypropylene fibers, preferred herein, the density is reported to be 910 kg/m³.

Maximum (largest) pore size and mean flow pore size determination:

The maximum pore size and mean flow pore size as used herein can be measured with a PMI Porometer in accordance with ASTM E1294-89 and F316-89 methods (capillary Constant as per ASTM method is 1; wetting fluid is Galwick, with Surface Tension of 15.9 mN/m; the surface tension of this fluid can be determined as set out herein below).
As Porometer a PMI Capillary Flow Porometry, model CFP-1200-AEX, serial number 01202004-1256 may suitably be used.
A wrinkle free, clean circular sample is obtained from the barrier component (which is free in the sample area of elastic material or film material, as described herein) or nonwoven barrier sheet (depending on which value needs to be tested in accord with the present disclosure), having a diameter of 1.0 cm (conditioned for 2 hours at 20 °C, 50% relative humidity). Using tweezers, the sample is immersed the in petri dish filled with the Galwick 15.9 mN/m wetting fluid such that the fluid completely covers the sample, for 30 seconds. Then the sample is turned, using tweezers, and re-immersed in the same dish and fluid, for a further 30 seconds. This ensures complete saturation of the pores with the wetting fluid.
Then, using tweezers, the saturated sample is directly placed onto the O-ring of the lower sample adaptor, without allowing the wetting fluid to drain, ensuring that that the O-ring is completely covered by the sample, but without covering the gauze during placement of the sample.
With the O-ring and sample facing upwards on the lower adopter and facing the upper adaptor, the Porometer is further prepared as per its manual and the measurement is started according to manual. The apparatus' software will analyse the measurements and report the maximum pore size as used herein. It will also calculate the mean flow pore size.

Hydrostatic head (Hydro Head):

The hydrostatic head (also referred to as hydro head) as used herein is measured with a low surface tension liquid, i.e. a 52 mN/m liquid (solution).

This liquid is prepared as set out below.

This test is performed as set out in co-pending application WO2005/112854A, conform the Inda/ Edana test WSP 80.6 (05). However, the water pressure (from below) is increased with a rate is 60 mbar/min.

A sample of 5 cm$^2$ is taken from the barrier component or nonwoven barrier sheet. The sample should be free from elastic material or edges that are connected to other materials.

The test head used has a 2.5 cm diameter; the protective sleeve used has a 2.2 cm diameter.

The test is performed on this sample and the Hydrostatic head value is obtained, and referred to herein.

52 mN/m (dynes/ cm) liquid preparation:

A 10 litre canister with tap is cleaned thoroughly 3 times with 2 litres polyethylene and then 3 times with 2 litres distilled/deionized water.

Then, it is filled with 10 litres distilled/deionized water and stirred with a clean stirring bar for 2 h, after which the water is released via the tap.

A 5 litre glass is cleaned 6 times with water and then 6 times with distilled/deionized water.

Then, 30.00 g of Na Cholate and 5 litres of distilled/deionized water are placed in the cleaned 5 litres glass. (NaCholate should have a TLC purity of >99%, e.g. supplied by Calbiochem, catalog # 229101). This is stirred with a clean stirring bar for about 5 min, until the Na Cholate is visibly dissolved.

The stirring bar is removed from the glass with a magnetic stick (without touching the solution) and then the Na cholate solution is poured into the 10 litres canister and more distilled/deionized water is added such that the concentration of the final solution is 3 g/l. This is further stirred with a stirring bar for 2 hours and then used.

This preparation of the solution and use thereof is at the temperature stated for the test for which it is used, or if no temperature is stated, it is kept at 20°C.

The surface tension of the solution is measured and this should be 52N/m. (The surface tension may be determined by method: ASTM D1331-56 ("Standard test method for surface and interfacial tension of solution of surface active agents") using a Kruss K12 tensiometer.)

Low surface tension Strike Through value method

The low surface tension strike through value referred to herein may be obtained by the Edana method WSP70.3 (05), except that a low surface tension liquid (see below) is used and a sample of 1 inch x 1 inch (25 mm x 25 mm) may be used. The sample should be free of elastic material or of edges that are connected to other materials.

The value obtained from this sample measurement is reported herein.

The low surface tension liquid is a liquid with a surface tension of 32 mN/m prepared as follows:
In a clean flask, 2.100 grams of Triton-X-100 is added to 500 ml distilled water (already in flask) and then 5000 ml distilled water is added. The solution is mixed for 30 minutes and then the surface tension is measured, which should be 32 mN/m.

(The surface tension may be determined by method: ASTM D1331-56 ("Standard test method for surface and interfacial tension of solution of surface active agents") using a Kruss K12 tensiometer.)

## Claims

1. An absorbent article comprising a barrier component which is part of a backsheet, or is on forms a side panel, a side flap, a waistband, a back ear or a cuff of said article and, which comprises a nonwoven barrier sheet, comprising at least a spunbond nonwoven web with spunbond fibers and at least a meltblown nonwoven web with meltblown fibers, **characterised in that** said spunbond fibers have a number average fiber diameter of from 10 to 15 microns, and said meltblown fibers have a number average fiber diameter from 1 to 5 $\mu$m, and the total weight percentage (by basis weight of the nonwoven barrier sheet) of the meltblown web(s) is from 5% to 30%, said nonwoven barrier sheet has a basis weigh from 10 to 45 g/m$^2$.

2. An absorbent article as in claim 1, whereby said nonwoven barrier sheet has pores of a largest pore size of less than 50 microns.

3. An absorbent article as in any of claims 1 or 2, whereby said meltblown fibers have an average fiber diameter 1 to 3 $\mu$m.

4. An absorbent article as in any of claims 1 to 3, whereby said nonwoven barrier sheet has pores of an average pore size of from 10 to 40 microns, and having at least 70% of the pores within +/- 25% of said average.

5. An absorbent article as in any of claims 1 to 4, whereby said nonwoven barrier sheet comprises as least 4 spunbond nonwoven webs and at least 4 meltblown nonwoven webs, preferably at least 5 meltblown nonwoven webs.

6. An absorbent article as in any of claims 1 to 5, whereby said nonwoven barrier sheet comprises at least two nonwoven layers that are partially attached to one another, preferably by less than 60% of the overlapping surface area, and each nonwoven layer comprises said at least one meltblown nonwoven web and said at least one spunbond nonwoven web.

7. An absorbent article an in any of claims 1 to 6, whereby said nonwoven barrier sheet has a Hydro Head value of at least 25 mbar, and preferably up to 45 mbar, and/ or a Low Surface Tension strike through value of at least 20 seconds, wherein the Hydro Head value and the Low-Surface Tension strike through value are determined with the methods disclosed in the specification.

8. An absorbent article as in any of claims 1 to 7, whereby said barrier component comprises an elastic laminate portion, comprising an elastic material, which is attached to said nonwoven barrier sheet, said barrier component preferably being part of or forming a cuff of said article.

9. An absorbent article as in claim 8, whereby said elastic laminate portion is patterned.

10. An absorbent article as in any of claims 1 to 9, whereby said barrier component comprises a skin care composition.

11. An absorbent article as in any of claims 1 to 10, whereby said barrier component comprises an ink composition.


**Patentansprüche**

1. Absorptionsartikel umfassend eine Sperrkomponente, die Teil einer Unterschicht ist oder ein Seitenfeld ist oder bildet, und eine Seitenklappe, ein Taillenband, eine hintere Lasche oder ein Bündchen des Artikels, und der eine Vliessperrschicht umfassend mindestens eine Spinnvliesbahn mit Spinnvliesfasern und mindestens eine schmelzgeblasene Vliesbahn mit schmelzgeblasenen Fasern umfasst, **dadurch gekennzeichnet, dass** die Spinnvliesfasern ein Zahlenmittel des Faserdurchmessers von 10 bis 15 $\mu$m aufweisen und die schmelzgeblasenen Fasern ein Zahlenmittel des Faserdurchmessers von 1 bis 5 $\mu$m aufweisen, und der Gesamtgewichtsprozentsatz (nach Basisgewicht der Vliessperrschicht) der schmelzgeblasene Bahn(en) von 5 % bis 30 % beträgt und die Vliessperrschicht ein Basisgewicht von 10 bis 45 g/m$^2$ aufweist.

2. Absorptionsartikel nach Anspruch 1, wobei die Vliessperrschicht Poren mit einer größten Porengröße von weniger als 50 $\mu$m aufweist.

3. Absorptionsartikel nach einem der Ansprüche 1 oder 2, wobei die schmelzgeblasenen Fasern einen durchschnittlichen Faserdurchmesser von 1 bis 3 $\mu$m aufweisen.

4. Absorptionsartikel nach einem der Ansprüche 1 bis 3, wobei die Vliessperrschicht Poren mit einer durchschnittlichen Porengröße von 10 bis 40 $\mu$m aufweist und zumindest 70 % der Poren innerhalb von +/- 25 % des Durchschnitts liegen.

5. Absorptionsartikel nach einem der Ansprüche 1 bis 4, wobei die Vliessperrschicht mindestens 4 Spinnvliesbahnen und mindestens 4 schmelzgeblasene Vliesbahnen, vorzugsweise mindestens 5 schmelzgeblasene Vliesbahnen umfasst.

6. Absorptionsartikel nach einem der Ansprüche 1 bis 5, wobei die Vliessperrschicht mindestens zwei Vliesschichten umfasst, die teilweise aneinander gebunden sind, vorzugsweise durch weniger als 60 % des überlappenden Oberflächenbereichs, und jede Vliesschicht die mindestens eine schmelzgeblasene Vliesbahn und die mindestens eine Spinnvliesbahn umfasst.

7. Absorptionsartikel nach einem der Ansprüche 1 bis 6, wobei die Vliessperrschicht einen Hydrohead-Wert von mindestens 25 mbar, und vorzugsweise bis zu 45 mbar, und/oder einen Durchschlagwert bei niedriger Oberflächenspannung von mindestens 20 Sekunden aufweist, wobei der Hydrohead-Wert und der Durchschlagwert bei niedriger Oberflächenspannung mit den in der Patentbeschreibung offenbarten Verfahren bestimmt werden.

**8.** Absorptionsartikel nach einem der Ansprüche 1 bis 7, wobei die Sperrkomponente einen elastischen Laminatabschnitt umfassend ein elastisches Material umfasst, der an die Vliessperrschicht gebunden ist, wobei die Sperrkomponente vorzugsweise Teil eines Bündchen des Artikel ist und ein Bündchen des Artikels bildet.

**9.** Absorptionsartikel nach Anspruch 8, wobei der elastische Laminatabschnitt gemustert ist.

**10.** Absorptionsartikel nach einem der Ansprüche 1 bis 9, wobei die Sperrkomponente eine Hautpflegezusammensetzung umfasst.

**11.** Absorptionsartikel nach einem der Ansprüche 1 bis 10, wobei die Sperrkomponente eine Tintenzusammensetzung umfasst.

**Revendications**

**1.** Article absorbant comprenant un composant barrière qui fait partie d'une feuille de fond, ou est ou forme un pan latéral, un rabat latéral, une bande de ceinture, une patte de dos ou un revers dudit article et qui comprend une feuille barrière non tissée, comprenant au moins une nappe non tissée filée-liée avec des fibres filées-liées et au moins une nappe non tissée soufflée en fusion avec des fibres soufflées en fusion, **caractérisé en ce que** lesdites fibres filées-liées ont un diamètre moyen de fibre en nombre allant de 10 à 15 micromètres, et lesdites fibres soufflées en fusion ont un diamètre moyen de fibre en nombre allant de 1 à 5 $\mu$m, et le pourcentage en poids total (en masse surfacique de la feuille barrière non tissée) de la ou des nappe(s) soufflée(s) en fusion va de 5 % à 30 %, et ladite feuille barrière non tissée a une masse surfacique allant de 10 à 45 g/m$^2$.

**2.** Article absorbant selon la revendication 1, selon lequel ladite feuille barrière non tissée a des pores d'une grosseur maximale de pores inférieure à 50 micromètres.

**3.** Article absorbant selon l'une quelconque des revendications 1 ou 2, selon lequel lesdites fibres soufflées en fusion ont un diamètre moyen de fibre de 1 à 3 $\mu$m.

**4.** Article absorbant selon l'une quelconque des revendications 1 à 3, selon lequel ladite feuille barrière non tissée a des pores d'une grosseur moyenne de pores allant de 10 à 40 micromètres, et ayant au moins 70 % des pores à +/-25 % de ladite moyenne.

**5.** Article absorbant selon l'une quelconque des revendications 1 à 4, selon lequel ladite feuille barrière non tissée comprend au moins 4 nappes non tissées filées-liées et au moins 4 nappes non tissées soufflées en fusion, de préférence au moins 5 nappes non tissées soufflées en fusion.

**6.** Article absorbant selon l'une quelconque des revendications 1 à 5, selon lequel ladite feuille barrière non tissée comprend au moins deux couches non tissées qui sont partiellement fixées l'une à l'autre, de préférence sur moins de 60 % de la superficie se chevauchant, et chaque couche non tissée comprend ladite au moins une nappe non tissée soufflée en fusion et ladite au moins une nappe non tissée filée-liée.

**7.** Article absorbant selon l'une quelconque des revendications 1 à 6, selon lequel ladite feuille barrière non tissée a une valeur de pression hydrostatique d'au moins 25 mbar, et de préférence jusqu'à 45 mbar, et/ou une valeur de transpercement à faible tension superficielle d'au moins 20 secondes, dans lequel la valeur de pression hydrostatique et la valeur de transpercement à faible tension superficielle sont déterminées avec les procédés décrits dans la description.

**8.** Article absorbant selon l'une quelconque des revendications 1 à 7, selon lequel ledit composant barrière comprend une partie stratifiée élastique, comprenant un matériau élastique, qui est fixé à ladite feuille barrière non tissée, ledit composant barrière faisant de préférence partie de ou formant un revers dudit article.

**9.** Article absorbant selon la revendication 8, selon lequel ladite partie stratifiée élastique est à motifs.

**10.** Article absorbant selon l'une quelconque des revendications 1 à 9, selon lequel ledit composant barrière comprend une composition de soin de la peau.

**11.** Article absorbant selon l'une quelconque des revendications 1 à 10, selon lequel ledit composant barrière comprend une composition d'encre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1417945 A
- WO 0037723 A2
- WO 0212601 A1
- WO 0229145 A2
- US 5607760 A
- US 5609587 A
- US 5635191 A
- US 5643588 A
- WO 9524173 A
- WO 2005112854 A